# EUROPÄISCHE PATENTANMELDUNG

(11) **EP 2 913 035 A1**
(43) Veröffentlichungstag der Anmeldung: **02.09.2015**
(21) Anmeldenummer: 14156971.5
(22) Anmeldetag: 27.02.2014
(51) Int. Cl.: A61F 9/007

(54) **Augenoperationsgerät mit einem chirurgischen Handstück und Steuerungsmitteln zum Steuern des Hubs der Hohlnadel**

(71) Anmelder: EOS GmbH, 52249 Eschweiler (DE)
(72) Erfinder: Klomp, Manfred, 4191AA Geldermalsen (NL)
(74) Vertreter: Schwarz & Partner

(57) **Zusammenfassung**

Die Erfindung betrifft ein Gerät mit einem chirurgischen Handstück (1), um eine Augenoperation an einem Patienten durchzuführen, wobei das chirurgische Handstück (1) einen Hohlzylinder (7) und eine in dem Hohlzylinder (7) verschiebbare Hohlnadel (10) aufweist, deren Stirnfläche (11) mit einer Öffnung (9) an der Mantelfläche des Hohlzylinders (7) zumindest beim Schließen der Öffnung (9) eine Schneide eines Messers zum Abtragen von biologischem Gewebe bildet, das durch einen Unterdruck in der Hohlnadel (10) durch die Hohlnadel (10) abgesaugt wird und, wobei das chirurgische Handstück (1) einen Antriebszylinder (14) aufweist, der durch einen Kolben (15) in zwei Zylinderhälften (16, 17) geteilt ist, und wobei Steuerungsmittel (5) des Geräts zum Ansteuern eines Druckluftgenerators (4) ausgebildet sind, um die zwei Zylinderhälften (16, 17) getrennt mit Druckluft (DL) und Gegendruckluft (GDL) zu beaufschlagen und die mit dem Kolben (15) verbundene Hohlnadel (10) zum Abtragen von biologischem Gewebe in eine erste Position (P1), in der die Öffnung (9) an der Mantelfläche des Hohlzylinders (7) geschlossen ist, und in eine zweite Position (P2), in der die Öffnung (9) an der Mantelfläche des Hohlzylinders (7) im Wesentlichen offen ist, anzutreiben, wobei die Steuerungsmittel (5) dazu ausgebildet sind die erste Zylinderhälfte (16) dauerhaft mit Druckluft (DL) zu beaufschlagen, um die Hohlnadel (10) in ihre erste Position (P1) zu positionieren, und die zweite Zylinderhälfte (17) für bestimmte Zeitperioden mit Gegendruckluft (GDL) zu beaufschlagen, um die Länge des Hubs (H1) der Hohlnadel (10) von der ersten Position (P1) in Richtung der zweiten Position (P2) und/oder, um die Dauer des Hubs (H1) der Hohlnadel (10) zu steuern.

## Beschreibung

Die Erfindung betrifft ein Gerät mit einem chirurgischen Handstück, um eine Augenoperation an einem Patienten durchzuführen, wobei das chirurgische Handstück einen Hohlzylinder und eine in dem Hohlzylinder verschiebbare Hohlnadel aufweist, deren Stirnfläche mit einer Öffnung an der Mantelfläche des Hohlzylinders zumindest beim Schließen der Öffnung eine Schneide eines Messers zum Abtragen von biologischem Gewebe bildet, das durch einen Unterdruck in der Hohlnadel durch die Hohlnadel abgesaugt wird und wobei das chirurgische Handstück einen Antriebszylinder aufweist, der durch einen Kolben in zwei Zylinderhälften geteilt ist, und wobei Steuerungsmittel des Geräts zum Ansteuern eines Druckluftgenerators ausgebildet sind, um die zwei Zylinderhälften getrennt mit Druckluft und Gegendruckluft zu beaufschlagen und die mit dem Kolben verbundene Hohlnadel zum Abtragen von biologischem Gewebe in eine erste Position, in der die Öffnung an der Mantelfläche des Hohlzylinders geschlossen ist, und in eine zweite Position, in der die Öffnung an der Mantelfläche des Hohlzylinders im Wesentlichen offen ist, anzutreiben.

Das Dokument US 2009/0281479 A1 offenbart so ein Augenoperationsgerät mit dem bei einer Vitrectomy-Operation der Glaskörper des Auges eines Patienten entfernt und durch eine ophthalmologische Spüllösung ersetzt wird. An das Augenoperationsgerät wird ein sogenanntes chirurgisches Handstück angeschlossen, mit dem das biologische Gewebe des Glaskörpers abgetragen und in eine Kassette des Augenoperationsgeräts abgesaugt wird. Das in dem Dokument US 2009/0281479 A1 offenbarte Handstück weist eine Hohlnadel auf, die in einem Hohlzylinder verschiebbar angeordnet ist und deren Stirnfläche mit einer Öffnung an der Mantelfläche des Hohlzylinders die Schneide eines Messers zum Abtragen des biologischen Gewebes bildet. Ein Unterdruckgenerator des Augenoperationsgeräts erzeugt den Unterdruck in der Hohlnadel zum Absaugen des abgetrennten biologischen Gewebes.

Ein pneumatischer Doppelzylinder, der durch eine Membran und eine Kolbenplatte in zwei Zylinderhälften geteilt ist, dient als translatorischer Antrieb der Hohlnadel in dem Hohlzylinder. Über ein Ventil wird abwechselnd einmal Druckluft in die eine Zylinderhälfte und dann wieder in die andere Zylinderhälfte geleitet, wodurch die Hohlnadel axial in dem Hohlzylinder verschoben wird, um beim Schließen der Öffnung durch die Hohlnadel biologisches Gewebe abzutragen. Zusätzlich ist in der einen Zylinderhälfte eine Spiralfeder vorgesehen, um die Hohlnadel in die zweite Position zurückzuziehen, in der die Öffnung an der Mantelfläche des Hohlzylinders offen ist.

Bei dem bekannten Handstück hat sich als Nachteil erwiesen, dass die Hohlnadel immer einen vollen Hub von der ersten Position, in der die Öffnung an der Mantelfläche des Hohlzylinders geschlossen ist, bis zu einem Anschlag in der zweiten Position der Hohlnadel durchführt. Hierdurch kommt es einerseits zu relativ wenigen Abtragvorgängen je Minute, da ein voller Hub von der ersten Position in die zweite Position und wieder zurück durchgeführt werden muss, damit die Schneide knapp vor Erreichen der ersten Position einen Schnitt durchführt und biologisches Gewebe abträgt. Dies kann allerdings nicht einfach dadurch verbessert werden, den Weg zwischen erster Position und zweiter Position zu verringern, da dann die Öffnung der Mantelfläche nur geringer geöffnet wird und auch nur weniger biologisches Gewebe in die Öffnung gedrückt und anschließend abgetragen wird. Eine größere Flexibilität für den Operateur, angepasst an das gemäß dem Operationsverlauf gerade abzutragende biologische Gewebe, wäre daher wünschenswert.

Als besonderer Nachteil des bekannten Handstücks hat sich weiters ergeben, dass bei jedem Zurückziehen der Hohlnadel in dem Hohlzylinder und somit bei jedem Öffnen der Öffnung in der Mantelfläche Spülflüssigkeit oder auch Umgebungsluft durch den Unterdruck in den Hohlzylinder gesaugt werden. Dies beeinflusst wiederum den tatsächlichen Unterdruck am Ende der Hohlnadel im Bereich der Öffnung an der Mantelfläche in dem Hohlzylinder, da der Unterdruckgenerator den vorgesehenen Unterdruck über die Ansaugleitung zur Kassette und die Hohlnadel bei raschen Druckänderungen im Bereich der Öffnung der Mantelfläche des Hohlzylinders nur relativ langsam in der Hohlnadel wieder herstellen kann. Hierdurch kann es kurzfristig zu einem zu geringen tatsächlichen Unterdruck an der Stirnfläche der Hohlnadel kommen, wodurch beim nächsten Hub abgetrenntes biologisches Gewebe nur schwach oder gar nicht abgesaugt wird. Wesentlich schlimmer ist es allerdings, wenn der tatsächliche Unterdruck an der Stirnfläche der Hohlnadel kurzfristig höher als vorgesehen ist, da es hierdurch zu gesundheitsschädlichen Ansaugvorgängen von noch nicht abgetrenntem biologischem Gewebe kommen kann. Das eingesaugte nicht abgetrennte biologische Gewebe verstopft dann erst recht die Hohlnadel, wodurch der tatsächliche Unterdruck am Ende der Hohlnadel noch weiter steigt und dem Auge irreparable Schäden zufügen kann.

Der Erfindung liegt die Aufgabe zugrunde ein Gerät mit einem chirurgischen Handstück und ein Verfahren zum Ansteuern eines Antriebszylinders eines chirurgischen Handstücks zu schaffen, bei dem die vorstehend angeführten Nachteile vermieden werden.

Erfindungsgemäß wird diese Aufgabenstellung bei einem Gerät mit einem chirurgischen Handstück dadurch gelöst, dass ....

Erfindungsgemäß wird diese Aufgabenstellung mit einem Verfahren zum Ansteuern eines Antriebszylinders des chirurgischen Handstücks gelöst bei dem folgende Verfahrensschritte vorgesehen sind...

Hierdurch ist der Vorteil erhalten, dass unterschiedliche Betriebsmodi zum Abtragen von biologischem Gewebe an dem Gerät eingestellt werden können, die durch die Steuermittel des Geräts ausschließlich durch Ansteuerung der Gegendruckluft des Antriebszylinders realisiert werden. Ein solcher Betriebsmodus könnte einen langen Hub vorsehen, bei dem die Hohlnadel den relativ langen Weg zwischen der ersten Position und der zweiten Position pendelt. Bei diesem Betriebsmodus wird die Öffnung der Mantelfläche weit geöffnet und viel biologisches Gewebe kann in die relativ große Öffnung hineinragen und wird beim Schließen der Öffnung durch die Hohlnadel abgetragen. Durch die große Öffnung dringt aber zusätzlich zu dem biologischen Gewebe auch relativ viel Spülflüssigkeit oder Umgebungsluft in die Hohlnadel ein, weshalb der tatsächliche Unterdruck an der Stirnfläche der Hohlnadel bei der Öffnung der Mantelfläche des Hohlzylinders bei jedem Hub zumindest kurzfristig relativ stark abnimmt.

Bei einem weiteren Betriebsmodus könnte die Hohlnadel in dem Hohlzylinder nur zwischen der ersten Position und einer Position, beispielsweise in der Mitte zwischen erster und zweiter Position, pendeln. Bei diesem weiteren Betriebsmodus wird nur ein Teil der Öffnung an der Mantelfläche des Hohlzylinders durch die Hohlnadel freigegeben, weshalb auch je Hub nur eine kleinere Mengen an biologischem Gewebe abgetragen werden kann und der tatsächliche Unterdruck am Ende der Hohlnadel bei jedem Hub kaum abnimmt und somit sehr stabil ist. Dies ist insbesondere deshalb wichtig, da neueste Forschungen ergeben haben, dass bei großen Druckunterschieden des Unterdrucks im Auge bei der Aspiration von abgeschnittenem biologischem Gewebe während der Operation etwa zwei bis drei Monate nach der Operation Löcher in der Retina entstehen, die einen Sehkraftverlust bewirken.

Mit den erfindungsgemäßen Steuerungsmitteln und gemäß dem erfindungsgemäßen

Verfahren zum Ansteuern des Antriebszylinders des chirurgischen Handstücks ist somit der Vorteil erhalten, dass der Operateur mit einem Fußschalter oder anderen Eingabemitteln des Geräts sehr flexibel der Situation angepasst den Hub der Hohlnadel verstellen kann, um die Schneidleistung des Handstücks einzustellen und den tatsächlichen Unterdruck am Ende der Hohlnadel im Bereich des Auges zu beeinflussen.

Weitere vorteilhafte Ausgestaltungen des erfindungsgemäßen Geräts werden im Folgenden anhand der Figuren näher erläutert.
Figur 1 zeigt ein Handstück eines Geräts zur Verwendung in der Augenchirurgie in einer Schnittdarstellung.
Figur 2 zeigt das Ende des Hohlzylinders samt Hohlnadel in der ersten Position in einer Schnittdarstellung.
Figur 3 zeigt das Ende des Hohlzylinders samt Hohlnadel in der zweiten Position in einer Schnittdarstellung.
Figur 4 zeigt den zeitlichen Ablauf relevanter Größen bei der Ansteuerung des Antriebszylinders des Handstücks.

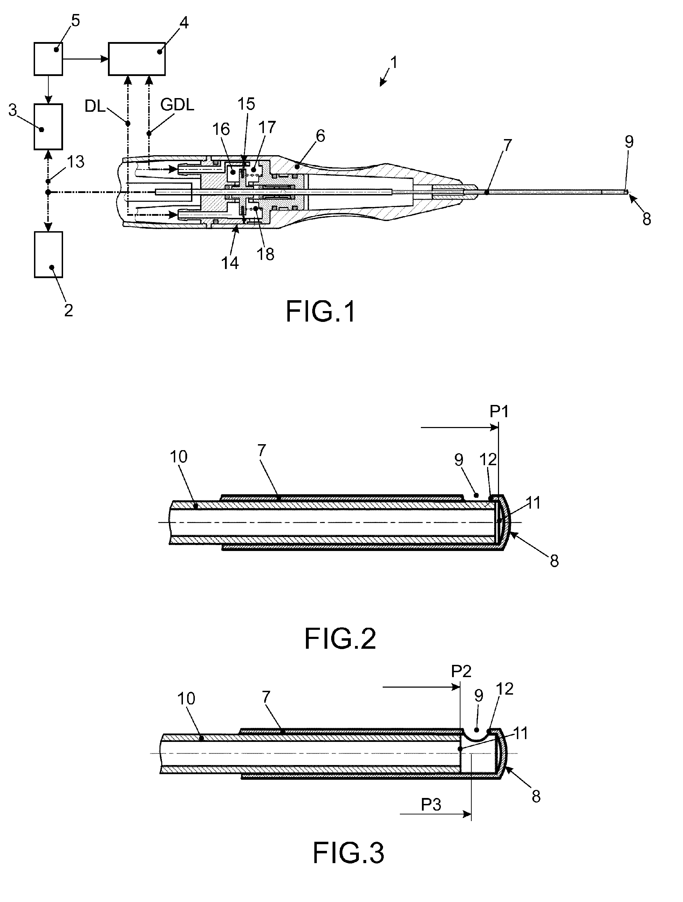

Figur 1 zeigt ein chirurgisches Handstück 1 eines Geräts, um eine Augenoperation an einem Patienten durchzuführen. Mit dem Gerät kann bei einer Vitrectomy-Operation der Glaskörper des Auges eines Patienten entfernt und durch eine ophthalmologische Spüllösung ersetzt werden. An das Gerät wird das Handstück 1 angeschlossen, mit dem das biologische Gewebe des Glaskörpers abgetragen und in eine Kassette 2 des Geräts abgesaugt wird. Erfindungswesentliche Elemente des Geräts sind in Figur 1 nur symbolisch als Unterdruckgenerator 3 zum Ansaugen abgetragenen biologischen Materials in die Kassette 2, als Druckluftgenerator 4 und Steuerungsmittel 5 dargestellt, worauf nachfolgend näher eingegangen ist.

Das chirurgische Handstück 1 weist einen Handgriff 6 auf, in dem ein Hohlzylinder 7 befestigt ist. Der Hohlzylinder 7 ist an seinem freien Ende 8 verschlossen und weist an seiner Mantelfläche im Bereich des freien Endes 8 eine Öffnung 9 auf. In dem Hohlzylinder 7 ist eine Hohlnadel 10 verschiebbar angeordnet, deren Ende eine Stirnfläche 11 bildet.

Figur 2 zeigt das freie Ende 8 des Hohlzylinders 7 in einer Schnittdarstellung, wobei die Hohlnadel 10 in Richtung von dem Handgriff 6 weg, ganz an das Ende 8 des Hohlzylinders 7 verschoben in einer ersten Position P1 dargestellt ist, in der die Öffnung 9 an der Mantelfläche des Hohlzylinders 7 durch die Hohlnadel 10 geschlossen ist. In Figur 3 ist die Hohlnadel 10 in Richtung des Handgriffs 6 verschoben, in der die Öffnung 9 an der Mantelfläche des Hohlzylinders 7 offen ist. Wenn die Hohlnadel 10 von ihrer zweiten Position P2 in ihre erste Position P1 verschoben wird, dann wird biologisches Gewebe des Auges, das in die Öffnung 9 an der Mantelfläche des Hohlzylinders 7 hineinragt, von der Stirnfläche 11 der Hohlnadel 10 gegen den Rand 12 der Öffnung 9 gedruckt und letztendlich abgetrennt beziehungsweise abgeschnitten.

Der Unterdruckgenerator 3 ist zum Erzeugen eines Unterdrucks in der Hohlnadel 10 zum Absaugen von abgetrenntem biologischem Gewebe vorgesehen und durch eine Venturipumpe gebildet. Der Operateur kann mit Hilfe eines Fußschalters und den Steuerungsmitteln 5 den Unterdruck für die Aspiration vorgeben. Der Unterdruckgenerator 3 ist über eine symbolisch dargestellte Anschlussleitung 13 mit einem Ende der Hohlnadel 10 verbunden, wodurch sich auch am anderen Ende der Hohlnadel 10 im Bereich der Stirnfläche 11 und im Bereich der Öffnung 9, die während der Operation unmittelbar im operierten Auge liegt, der von dem Operateur vorgegebene Unterdruck einstellt. Je nachdem, ob über die Öffnung 9 gerade biologisches Gewebe, Spülflüssigkeit oder Luft angesaugt wird und je nach aktueller Position P der Hohlnadel 10, ob die Öffnung 9 gerade von der Hohlnadel 10 verschlossen oder offen ist, stellt sich im Bereich der Öffnung 9 ein tatsächlicher Unterdruck ein, der kurzfristig über oder unter dem von dem Operateur vorgegebenen Unterdruck liegen kann.

Das chirurgische Handstück 1 weist einen Antriebszylinder 14 auf, der durch einen Kolben 15 in zwei Zylinderhälften 16 und 17 geteilt ist und als translatorischer Antrieb der Hohlnadel 10 in dem Hohlzylinder 7 dient. Der Kolben 15 ist durch eine Membran und eine Kolbenplatte gebildet. Die Steuerungsmittel 5 des Geräts sind zum Ansteuern des Druckluftgenerators 4 ausgebildet, um die zwei Zylinderhälften 16 und 17 getrennt mit Druckluft DL und Gegendruckluft GDL zu beaufschlagen und die mit dem Kolben 15 verbundene Hohlnadel 10 zum Abtragen von biologischem Gewebe in die erste Position P1 und in eine zweite Position P2 zu bewegen. Zusätzlich ist in der zweiten Zylinderhälfte 17 eine Spiralfeder 18 vorgesehen, um die Hohlnadel 10 in die zweite Position P2 zurückzuziehen, in der die Öffnung 9 an der Mantelfläche des Hohlzylinders 7 offen ist.

Die Steuerungsmittel 5 sind nunmehr dazu ausgebildet die erste Zylinderhälfte 16 dauerhaft mit Druckluft DL zu beaufschlagen, um die Hohlnadel 10 in ihre erste Position P1 zu positionieren. Weiters beaufschlagen die Steuerungsmittel 5 die zweite Zylinderhälfte 17 für bestimmte Zeitperioden mit der Gegendruckluft GDL, um die Länge des Hubs H der Hohlnadel 10 von der ersten Position P1 in Richtung der zweiten Position P2 und beziehungsweise oder, um die Dauer des Hubs H der Hohlnadel 10 zu steuern.

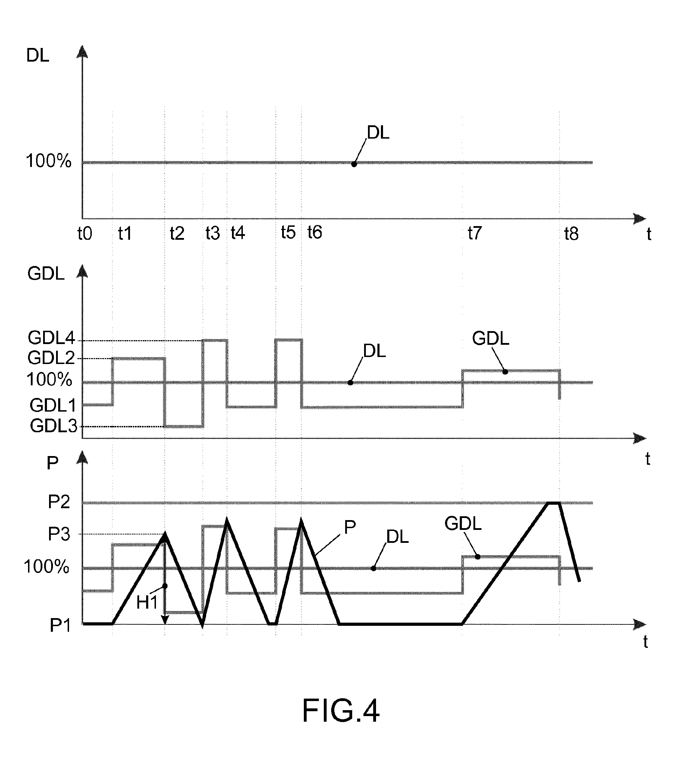

Figur 4 zeigt den zeitlichen Ablauf der Druckluft DL, der Gegendruckluft GDL und der daraus folgenden Position P der Stirnfläche 11 der Hohlnadel 10 in dem Hohlzylinder 7 je nach Ansteuerung des Antriebszylinders 14 durch die Steuermittel 5. Der Druckluftgenerator 4 beaufschlagt die erste Zylinderhälfte 16 dauerhaft während des gesamten in Figur 4 dargestellten Zeitverlaufs mit der Druckluft DL von beispielsweise einem Bar über dem atmosphärischen Druck.

Zu einem Zeitpunkt t0 beaufschlagt der Druckluftgenerator 4 die zweite Zylinderhälfte 17, gesteuert durch die Steuerungsmittel 5, mit einem unter dem Druck der Druckluft DL liegenden Druck der Gegendruckluft GDL1, weshalb sich die Hohlnadel 10 in der ersten Position P1 befindet und die Öffnung 9, wie in Figur 2 dargestellt, geschlossen ist. Zu einem Zeitpunkt t1 beaufschlagt der Druckluftgenerator 4 die zweite Zylinderhälfte 17 mit einem über dem Druck der Druckluft DL liegenden Druck der Gegendruckluft GDL2, weshalb sich die Hohlnadel 10 von der ersten Position P1 in Richtung der zweiten Position P2 bewegt. Die Geschwindigkeit der Hohlnadel 10 hängt hierbei vom Differenzdruck Gegenluftdruck GLD2 minus Druckluft DL ab und ist in Figur 4 an der Steigung der Geraden der Position P vom Zeitpunkt t1 bis zu einem Zeitpunkt t2 erkennbar.

Zu dem Zeitpunkt t2 hat die Stirnfläche 11 der Hohlnadel 10 eine dritte Position P3 in dem Hohlzylinder 7 erreicht und die Öffnung 9 ist teilweise geöffnet. Von der ersten Position P1 bis zur dritten Position P3 hat die Hohlnadel 10 einen ersten Hub H1 während einer Zeitdauer von t2 minus t1 durchgeführt. Zu dem Zeitpunkt t2 beaufschlagt der Druckluftgenerator 4 die zweite Zylinderhälfte 17 mit einem unter dem Druck der Druckluft DL liegenden Druck der Gegendruckluft GDL3, weshalb sich die Hohlnadel 10 von der dritten Position P3 in Richtung der ersten Position P1 bewegt, bis sie diese zu einem Zeitpunkt t3 erreicht.

Anhand der vorstehenden Beschreibung ist ersichtlich, dass der Operateur mit dem Fußschalter, oder anderen Eingabemitteln des Geräts, mittels der Steuereinrichtung 5 die Länge des Hubs H und die Dauer des Hubs H der Hohlnadel 10 steuern kann. Wie vorstehend beschrieben stellt sich im Bereich der Öffnung 9 ein tatsächlicher Unterdruck ein, der im Wesentlichen dem vom Unterdruckgenerator 3 vorgegebenen Unterdruck entspricht, aber je nach Stellung der Hohlnadel 10 (Öffnung 9 geöffnet oder geschlossen) und je nach gerade in der Hohlnadel 10 abgesaugtem Material kurzfristig auch wesentlich davon abweichen kann.

Die Steuerungsmittel sind nunmehr zur Steuerung des tatsächlichen Unterdrucks an der Stirnfläche 11 der Hohlnadel 10 dazu ausgebildet sowohl den Unterdruckgenerator 3 zu steuern, als auch die Länge und die Dauer des Hubs H der Hohlnadel 10 zu berücksichtigen beziehungsweise diese verändern, um den tatsächlichen Unterdruck an der Stirnfläche 11 der Hohlnadel 10 zu beeinflussen. Hierdurch ist der Vorteil erhalten, dass der Operateur mit dem Fußschalter oder anderen Eingabemitteln des Geräts sehr flexibel und der Situation angepasst den Hub H der Hohlnadel 10 verstellen kann, um die Schneidleistung des Handstücks einzustellen und den tatsächlichen Unterdruck am Ende der Hohlnadel im Bereich des Auges zu beeinflussen.

Weiters ist der Vorteil erhalten, dass der Operateur den Hub H der Hohlnadel 10 klein einstellen kann, um mit relativ häufigen Schnitten jeweils kleine Mengen an biologischem Material abzutrennen, oder den Hub H von der Position P1 bis zur Position P2 relativ groß einstellen kann, um mit weniger Schnitten relativ große Abschnitte biologischem Materials abzuschneiden.

In dem Ausführungsbeispiel gemäß Figur 4 wurde ein sich ständig in seiner Länge und Zeitdauer ändernder Hub dargestellt, um die Vielfalt der Steuerungsmöglichkeiten aufzuzeigen. In der Praxis und während einer Operation wird das Handstück aber eine Vielzahl gleicher Hübe H ausführen, bis der Operateur durch Änderung der Fußstellung des Fußschalters zur Änderung des Schnittmodus verändert. Eine kurzfristige Änderung, gegebenenfalls auch nur für einen Hub, können die Steuerungsmittel 5 automatisch durchführen, wenn anhand einer Druckleistungsänderung des Druckluftgenerators 4 detektiert wird, dass die Hohlnadel 10 verstopft ist oder Luft angesaugt hat. In diesem Fall kann der Hohlzylinder 10 so gesteuert werden, dass die Öffnung 9 länger oder kürzer offen bleibt, um einen zu niedrigen und insbesondere einen zu hohen tatsächlichen Unterdruck an der Öffnung 9 zu vermeiden.

Anhand der vorstehenden Beschreibung ist auch das Verfahren zum Ansteuern des Antriebszylinders 14 des chirurgischen Handstücks 1 mit Druckluft DL und Gegenduckluft GDL, um die in dem Hohlzylinder 7 verschiebbar angeordneten Hohlnadel 10 anzutreiben und biologisches Gewebe an einer Schneide abzutragen offenbart. Bei diesem Verfahren werden die Folgenden Verfahressschritte durchgeführt:
- Dauerhaftes Beaufschlagen einer ersten Zylinderhälfte 16 des Antriebszylinders 14 mit Druckluft DL, um die Hohlnadel 10 in die erste Position P1 zu verschieben, in der die Hohlnadel 10 die an der Mantelfläche des Hohlzylinders 7 vorgesehene Öffnung 9 verschließt;
- Beaufschlagen der zweiten Zylinderhälfte 17 des Antriebszylinders 14 mit Gegendruckluft GDL während bestimmter Zeitperioden, um die Hohlnadel 10 in Richtung einer zweiten Position P2 zu verschieben, in der die Hohlnadel 10 die Öffnung 9 an der Mantelfläche des Hohlzylinders 1 im Wesentlichen freigibt und, um die Länge des Hubs H der Hohlnadel 10 von der ersten Position P1 in Richtung der zweiten Position P2 und/oder, um die Dauer des Hubs H der Hohlnadel 10 zu steuern.

## Patentansprüche

1. Gerät mit einem chirurgischen Handstück (1), um eine Augenoperation an einem Patienten durchzuführen, wobei das chirurgische Handstück (1) einen Hohlzylinder (7) und eine in dem Hohlzylinder (7) verschiebbare Hohlnadel (10) aufweist, deren Stirnfläche (11) mit einer Öffnung (9) an der Mantelfläche des Hohlzylinders (7) zumindest beim Schließen der Öffnung (9) eine Schneide eines Messers zum Abtragen von biologischem Gewebe bildet, das durch einen Unterdruck in der Hohlnadel (10) durch die Hohlnadel (10) abgesaugt wird und, wobei das chirurgische Handstück (1) einen Antriebszylinder (14) aufweist, der durch einen Kolben (15) in zwei Zylinderhälften (16, 17) geteilt ist, und wobei Steuerungsmittel (5) des Geräts zum Ansteuern eines Druckluftgenerators (4) ausgebildet sind, um die zwei Zylinderhälften (16, 17) getrennt mit Druckluft (DL) und Gegendruckluft (GDL) zu beaufschlagen und die mit dem Kolben (15) verbundene Hohlnadel (10) zum Abtragen von biologischem Gewebe in eine erste Position (P1), in der die Öffnung (9) an der Mantelfläche des Hohlzylinders (7) geschlossen ist, und in eine zweite Position (P2), in der die Öffnung (9) an der Mantelfläche des Hohlzylinders (7) im Wesentlichen offen ist, anzutreiben, **dadurch gekennzeichnet, dass**
die Steuerungsmittel (5) dazu ausgebildet sind die erste Zylinderhälfte (16) dauerhaft mit Druckluft (DL) zu beaufschlagen, um die Hohlnadel (10) in ihre erste Position (P1) zu positionieren, und die zweite Zylinderhälfte (17) für bestimmte Zeitperioden mit Gegendruckluft (GDL) zu beaufschlagen, um die Länge des Hubs (H1) der Hohlnadel (10) von der ersten Position (P1) in Richtung der zweiten Position (P2) und/oder, um die Dauer des Hubs (H1) der Hohlnadel (10) zu steuern.

2. Gerät gemäß Anspruch 1, **dadurch gekennzeichnet, dass** ein Unterdruckgenerator (3) zum Erzeugen des Unterdrucks in der Hohlnadel (10) zum Absaugen von abgetragenem biologischen Gewebe vorgesehen ist und, dass die Steuerungsmittel (5) zum Steuern des Unterdrucks in der Hohlnadel (10) auch die Länge und die Dauer des Hubs (H1) der Hohlnadel (10) berücksichtigen beziehungsweise diese verändern, um den tatsächlichen Unterdruck an der Stirnfläche (11) der Hohlnadel (10) zu beeinflussen.

3. Gerät gemäß Anspruch 2, **dadurch gekennzeichnet, dass** der Unterdruckgenerator (3) durch eine Venturipumpe gebildet ist.

4. Gerät gemäß einem der vorherigen Ansprüche, **dadurch gekennzeichnet, dass** der Druckluftgenerator (4) zum Erzeugen eines den Druck der Druckluft (DL) für die erste Zylinderhälfte (16) übersteigenden Drucks der Gegendruckluft (GDL) für die zweite Zylinderhälfte (17) ausgebildet ist.

5. Gerät gemäß einem der vorherigen Ansprüche, **dadurch gekennzeichnet, dass** die Steuerungsmittel (5) zum Ansteuern des Druckluftgenerators (4) ausgebildet sind, um die Stärke der Gegendruckluft (GDL) während der bestimmten Zeitperioden zu verändern.

6. Verfahren zum Ansteuern eines Antriebszylinders (14) eines chirurgischen Handstücks (1) mit Druckluft (DL) und Gegenduckluft (GDL), um eine in einem Hohlzylinder (7) verschiebbar angeordneten Hohlnadel (10) anzutreiben und biologisches Gewebe an einer Schneide abzutragen, **dadurch gekennzeichnet, dass** folgende Verfahressschritte durchgeführt werden:
• Dauerhaftes Beaufschlagen einer ersten Zylinderhälfte (16) des Antriebszylinders (14) mit Druckluft (DL), um die Hohlnadel (10) in eine erste Position (P1) zu verschieben, in der die Hohlnadel (10) eine an der Mantelfläche des Hohlzylinders (7) vorgesehene Öffnung (9) verschließt;
• Beaufschlagen einer zweiten Zylinderhälfte (17) des Antriebszylinders (14) mit Gegendruckluft (GDL) während bestimmter Zeitperioden, um die Hohlnadel (10) in Richtung einer zweiten Position (P2) zu verschieben, in der die Hohlnadel (10) die Öffnung (9) an der Mantelfläche des Hohlzylinders (7) im Wesentlichen freigibt und, um die Länge des Hubs (H1) der Hohlnadel (10) von der ersten Position (P1) in Richtung der zweiten Position (P2) und/oder, um die Dauer des Hubs (H1) der Hohlnadel (10) zu steuern.

7. Verfahren gemäß Anspruch 6, **dadurch gekennzeichnet, dass** die zweite Zylinderhälfte (17) des Antriebszylinders (14) derart mit Gegendruckluft (GDL) beaufschlagt wird, um den Unterdruck in dem Hohlzylinder (7) durch die Länge und Dauer des Hubs (H1) der Hohlnadel (10) zu steuern.
